# EUROPEAN PATENT APPLICATION

(11) **EP 0 983 763 A1**
(43) Date of publication of application: **08.03.2000**
(21) Application number: 98911141.4
(22) Date of filing: 31.03.1998
(51) Int. Cl.: A61K 31/165

(54) **PREVENTIVES FOR RECONSTRICTION**

(30) Priority: 01.04.1997 JP 8264997
(71) Applicant: TAISHO PHARMACEUTICAL CO. LTD, Tokyo 170-8633 (JP)
(72) Inventor: ASAMI, Yumiko Taisho Pharmaceutical Co., Ltd., Tokyo 170-8633 (JP); AKIYOSHI, Kazuhiko Taisho Pharmaceutical Co., Ltd., Tokyo 170-8633 (JP); YAMAGISHI, Izumi Taisho Pharmaceutical Co., Ltd., Toshima-ku Tokyo 170-8633 (JP); TOMOIKE, Hideki Taisho Pharmaceutical Co., Ltd., Toshima-ku Tokyo 170-8633 (JP)
(74) Representative: Albrecht, Thomas, Dr.
(86) International application number: JP9801486
(87) International publication number: WO9843626

(57) **Abstract**

This invention relates to an agent for preventing restenosis after coronary intervention therapy which comprises as an active ingredient a compound represented by the formula

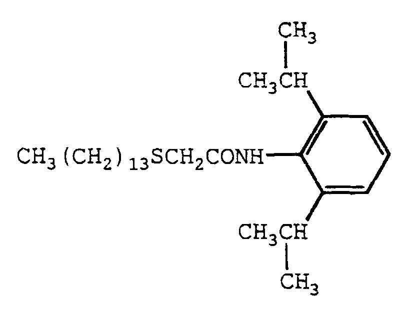

The agent for preventing restenosis according to the invention can provide a new use for preventing restenosis after coronary intervention therapy for the patients suffering from ischemic heart diseases who have severe stenosis or cannot be treated by a pharmacotherapy among those having a seizure of angina pectoris caused by stenosis of the coronary artery.

## Description

### TECHNICAL FIELD

This invention relates to an agent for preventing restenosis after coronary intervention.

Coronary intervention is a therapy for angina pectoris by means of the reconstructive surgery for blood circulation of the coronary artery. This therapy may be represented by a PTCA (Percutaneous Transluminal Coronary Angioplasty) therapy in which a highly intense organic stenosis manifested in the coronary artery is physically enlarged by means of a balloon catheter inserted into the blood vessel, a stent therapy in which a metal support stent is embedded from the inside after enlarged with a balloon, an atherectomy therapy in which atheroma is excised, etc., and recently, it has been positively performed on the patients suffering from ischemic heart diseases such as angina pectoris and myocardial infarction, in addition to a pharmacotherapy.

### BACKGROUND ART

Pathologenically, neogenetic intimal thickening sites after restenosis, which may somewhat vary depending upon the constitution of the initial intimal thickening, comprises mainly smooth muscle cells transformed into a synthetic type, and additionally macrophages and neogenetic blood vessels.

More specifically, it is considered that intimal thickening is caused by the abnormally promoted migration and proliferation of smooth muscle cells owing to the blood vessel-repairing function of a living body or, in addition, by the inhibition of apotosis which is a controlling mechanism of the growth of the smooth muscles. However, the details thereof have not been elucidated yet.

Many attempts to develop a drug have been hitherto made by means of an in vitro screening of an inhibiting activity on the growth of the smooth muscles, but no product has been yet proved to be efficacious in patients or marketed as a drug either in Japan or in any other country.

### DISCLOSURE OF INVENTION

Of the coronary intervention therapies, the PTCA has been most frequently practiced now and performed on the patients suffering from ischemic heart diseases who have severe stenosis or cannot be treated by a pharmacotherapy among those having a seizure of angina pectoris caused by stenosis of the coronary artery. With the recent improvement in the technique thereof, the number of patients treated has increased.

The problem of the PTCA is restenosis. This is the condition that, even when the stenotic site in the blood vessel is physically enlarged by the PTCA, stenosis may develop again at the rate of 35-40% usually within 3 months after the treatment. It is said that the restenosis is based upon the elastic recoil which may develop at an early stage after the PTCA, thrombus formation, and the intimal thickening and remodeling of the coronary artery which may develop 1-3 months after the treatment, or the like.

Of these problems, the most clinically serious problem is the restenosis caused by the intimal thickening which may develop 1-3 months after the PTCA. Under present conditions, no drug can inhibit the intimal thickening.

In view of the above problem, the present inventors have conducted a screening with a model for thickening of the intima by endothelial injury in a rabbit, and as a result, have found out an effective compound, upon which this invention has been completed.

More specifically, this invention is directed to an agent for preventing restenosis after coronary intervention which comprises as an active ingredient a compound represented by the formula

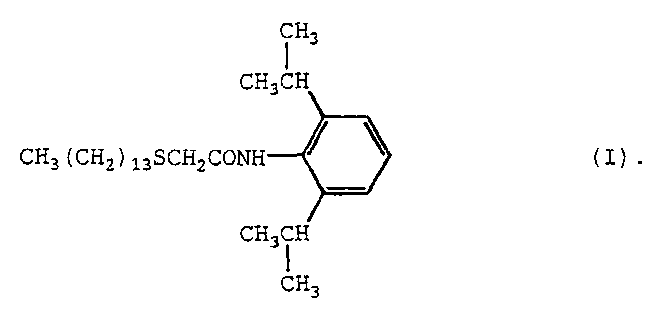

The compound represented by the formula (I) (hereinafter referred to as the compound of the invention) showed a potent inhibiting activity on intimal thickening in two types of the models for thickening of the intima caused by endothelial injury in rabbits.

The model for thickening of the intima caused by endothelial injury in normal rabbits is the model in which thickening of the intima is induced by the migration of the medium smooth muscle to the intima thereof and the proliferation of the intimal smooth muscle of a synthetic type by peeling off the intima of the blood vessel, and it has been well-known as a model for human restenosis after the PTCA.

The other model, in which the supply of cholesterol by denatured low density lipoprotein (LDL) is accelerated in the intima due to endothelial injury in hyperlipemic WHHL rabbits, is regarded as more approximate to a human pathological model.

Since the compound of the invention could inhibit intimal thickening of the artery at such a low dose as not to change a serum cholesterol level in two models, the compound is considered to act directly on the arterial wall.

As the mechanism of the inhibition of intimal thickening by the compound of the invention, an ACAT inhibiting activity is partially considered to improve in cholesterol metabolism in the arterial wall. However, an ACAT inhibitor shows an improved cholesterol metabolism mainly in macrophages (Thomas M.A. Bocan et al., Arteriosclerosis and Thrombosis, Vol. 11, pp. 1830-1843 (1991)) and is thought to be hardly effective on the lesion mainly of the smooth muscle.

On the other hand, since the compound of the invention highly inhibited the intimal thickening mainly of the smooth muscle, irrespective of the presence of the deposited cholesterol, other mechanism of action than an ACAT inhibitor is believed to be involved.

The compound of the invention is expected to have an antioxidant activity, because the sulfur in its structural formula is oxidized. It is reported that antioxidant substances such as tocopherol, probucol, etc. could secondarily inhibit the proliferation of smooth muscle cells via the inhibiting activity on the secretion of interleukin-1 (Ann L. Akeson et al., Atherosclerosis, Vol. 86, pp. 261-270 (1991)). Accordingly, there is also a possibility that an antioxidant activity may participate in the inhibiting activity of the intimal thickening by the compound of the invention. In any case, since the compound of the invention was histopathologically observed to decrease the number of smooth muscle cells during the proliferation period in the thickened intima, it is apparent that the compound of the invention can possess an inhibiting activity on abnormal proliferation of smooth muscle cells after injury of the blood vessel according to some mechanism of action.

The compound of the invention is a well-known compound as disclosed in U.S. Patent No. 5,475,130.

### BEST MODE FOR CARRYING OUT THE INVENTION

A dose of the compound which is an active ingredient in the invention may vary depending upon the conditions. A daily dose for adults is usually 0.5-120 mg/human in the case of oral administration and it may be administered once per day or in several divided doses per day.

The preventive agent of the invention may be applied after prepared in a solid form such as tablets, pills, capsules, granules, dry syrups, etc.

In preparing a solid preparation, various additives may be used, for example, excipients, disintegrators, binders, lubricants or coating bases, and an agitation granulation method, a fluidized bed granulation method or a fracture granulation method can be employed.

The excipients may include, for example, mannitol, xylitol, sorbitol, glucose, sucrose, lactose, crystalline cellulose, crystalline cellulose·carboxymethylcellulose sodium, calcium hydrogenphosphate, wheat starch, rice starch, corn starch, potato starch, carboxymethylstarch sodium, dextrin, α-cyclodextrin, β-cyclodextrin, a carboxyvinyl polymer, light anhydrous silicic acid, titanium oxide, magnesium metasilicate aluminate, polyethylene glycol, a middle chain fatty acid triglyceride, etc.

The disintegrators may include a hydroxypropyl cellulose with a low degree of substitution, carboxymethylcellulose, carboxymethyl-cellulose calcium, carboxymethylcellulose sodium, croscarmellose sodium·A-type (ACTISOL), starch, crystalline cellulose, hydroxypropyl starch, partially pregelatinized starch, etc.

The binders may include, for example, methylcellulose, hydroxypropylcellulose, hydroxypropylmethylcellulose, polyvinyl pyrrolidone, gelatin, gum arabic, ethylcellulose, polyvinyl alcohol, pullulan, pregelatinized starch, agar, tragacanth, sodium alginate, alginic acid propylene glycol ester, etc.

The lubricants may include, for example, stearic acid, magnesium stearate, calcium stearate, polyoxyl stearate, cetanol, talc, hardened oil, sucrose fatty acid ester, dimethylpolysiloxane, microcrystalline wax, beeswax, bleached beeswax, etc.

The antioxidants may include, for example, dibutylhydroxytoluene (BHT), pyrogallic acid propyl ester, butylhydroxyanisole (BHA), α-tocopherol, citric acid, etc.

The coating agents may include, for example, hydroxypropylmethylcellulose, hydroxypropylcellulose, methylcellulose, ethylcellulose, hydroxypropylmethylcellulose phthalate, hydroxypropylmethylcellulose acetate succinate, carboxymethylethylcellulose, cellulose acetate phthalate, polyvinyl acetal diethylaminoacetate, an aminoalkylmethacrylate copolymer, a methacrylic copolymer, cellulose acetate trimellitate (CAT), polyvinyl acetate phthalate, shellac, etc.

The coloring agents may include, for example, a tar color, titanium oxide, etc.

The corrigents may include citric acid, adipic acid, ascorbic acid, menthol, etc.

The surfactants may include, for example, polyoxyethylene hardened castor oil, glycerol monostearate, sorbitan monostearate, sorbitan monopalmitate, sorbitan monolaurate, a polyoxyethylene-polyoxypropylene block copolymer, polysorbates, sodium lauryl sulfate, Macrogols, a sucrose fatty acid ester, etc.

The plasticizers may include triethyl citrate, triacetin, cetanol, etc.

The preventive agent of the invention may be applied orally or parenterally in any other form suitable for each administration. Examples of such forms are solutions, emulsions, suppositories, etc.

### INDUSTRIAL APPLICABILITY

The compound of the invention showed an equal or higher inhibiting activity on intimal thickening at an extremely lower dose of 1/500 to 1/1000 or less, as compared with the two known agents in a model for thickening of the intima by vascular endothelial injury which is an animal model for human restenosis after the PTCA, and can be a highly effective agent for preventing the restenosis after the PTCA which has not been clinically elucidated yet.

This invention will be more fully explained by way of the following Test Examples and Examples.

### Test Example 1 [Inhibiting activity on intimal thickening in a model for vascular endothelial injury in normal rabbits]

### (Test Procedure)

As test animals were used a group of 5 NZW strain male rabbits of 2 months (available from Kitayama RABES K.K.) After a drug was administered to rabbits for 1 week, a balloon catheter for removing arterial embolus (Fogarty catheter, E-060-2F, available from Baxter Co., Inc.) was inserted into the right common carotid artery via the right external carotid artery of the rabbits under anesthesia with pentobarbital. The right common carotid artery was abraded three times with the balloon swollen (a balloon pressure of 0.5-1.0 atm) to produce a model for the injured vascular endothelium. The drug was administered for 2 weeks after the endothelium injury, and then the artery was excised and fixed with 10% neutral buffered formalin to prepare a slice of the arterial ring tissue. Elastic fiber-stained tissue preparations were prepared by Elastic Van Gieson (EVG) staining. Areas of the intima (I) and of the medium (M) were measured by means of an image analysis system (Adobe Photoshop, Unitimage for the Apple Macintosh) and a ratio of both values (I/M) was calculated to determine a level of intimal thickening.

The drug was given in admixture with a rabbit feed RC-4 (available from Oriental Kobo K.K.) at the concentration of 0.0003% and a dose was calculated from the amount of feed ingested and the body weight. As a control drug was used a promoting agent for keloid healing, tranilast, which was already known to possess an inhibiting activity for intimal thickening, in admixture with the RC-4 at the concentration of 0.167%.

### (Results)

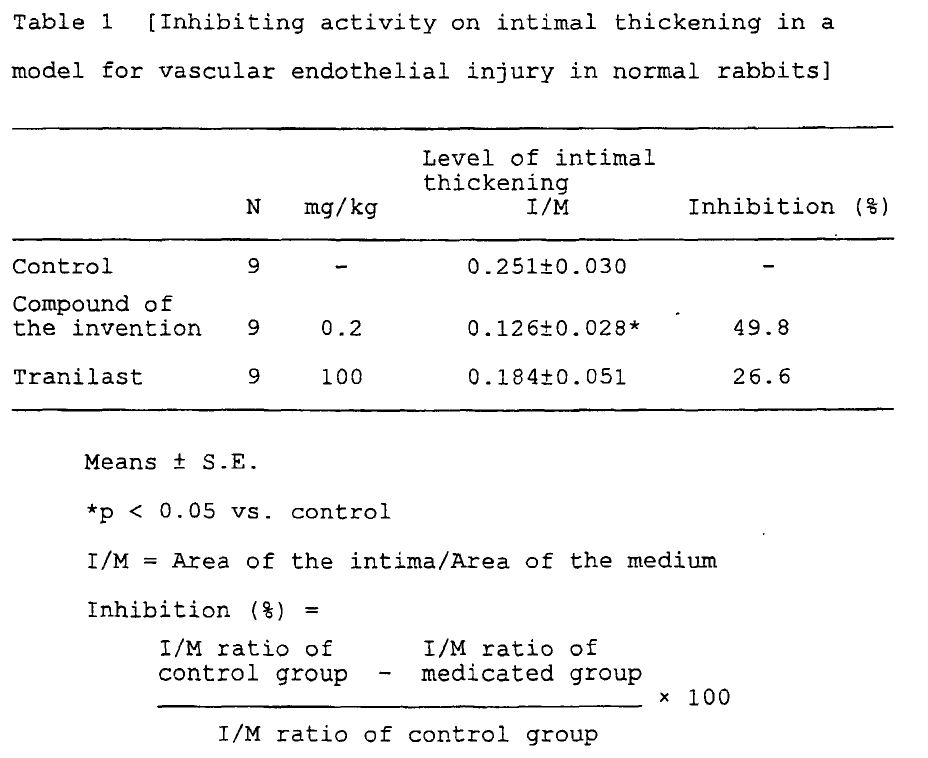

### Test Example 2 [Inhibiting activity on intimal thickening in a model for vascular endothelial injury in WHHL rabbits]

### (Test Procedure)

As test animals were used a group of 4 homozygous hereditary hyperlipemic WHHL strain male rabbits of 2 months (available from Kitayama RABES K.K.) A balloon catheter for removing arterial embolus (Fogarty catheter, E-060-2F, available from Baxter Co., Inc.) was inserted into the right common carotid artery via the right external carotid artery of the rabbits under anesthesia with pentobarbital. The right common carotid artery was abraded three times with the balloon swollen (a balloon pressure of 0.5-1.0 atm) to produce a model for the injured vascular endothelium. The drug was administered for 4 weeks after the endothelium injury, and then the artery was excised and fixed with 10% neutral buffered formalin to prepare a slice of the arterial ring tissue. Elastic fiber-stained tissue preparations were prepared by Elastic Van Gieson (EVG) staining. Areas of the intima (I) and of the medium (M) were measured by means of an image analysis system (Adobe Photoshop, Unitimage for the Apple Macintosh) and a ratio of both values (I/M) was calculated to determine a level of intimal thickening. A total blood cholesterol level was measured according to an enzyme method (Autosera CHO-2, Daiichi Kagaku Yakuhin K.K.) using an autoanalyzer (Hitachi 7150 type).

The drug was given in admixture with a rabbit feed RC-4 (available from Oriental Kobo K.K.) at the concentrations of 0.0003% and of 0.003%, and then a dose was calculated from the amount of feed ingested and the body weight. As a control drug was used the antihyperlipemic agent, probucol, which was already known to possess an inhibiting activity for intimal thickening in a hyperlipemic model, in admixture with the RC-4 at the concentration of 0.35%.

### (Results)

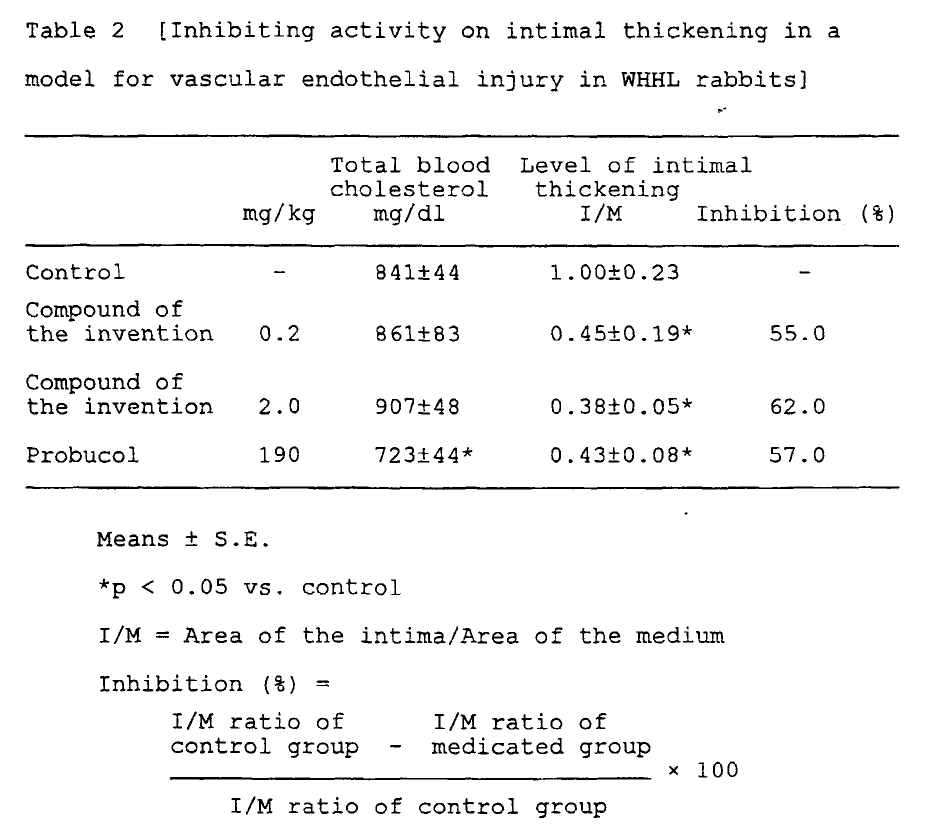

### Example 1

In 945 g of a middle chain fatty acid triglyceride and 5 g of Polysorbate 80 was dissolved 50 g of the compound of the invention to prepare a solution for encapsulation.

### Example 2

In 895 g of a middle chain fatty acid triglyceride and 5 g of Polysorbate 80 was dissolved 100 g of the compound of the invention to prepare a solution for encapsulation.

### Example 3

In 985 g of soybean oil and 5 g of Polysorbate 80 was dissolved 10 g of the compound of the invention to prepare a solution for encapsulation.

### Example 4

In 945 g of a middle chain fatty acid triglyceride and 5 g of the Polysorbate was dissolved 50 g of the compound of the invention. 10 g of the resulting solution was adsorbed into a premix of 20 g of calcium silicate and 40 g of calcium hydrogenphosphate and then 29 g of mannitol was added to prepare a powdery mixture. 1 g of magnesium stearate was further added to prepare powders.

### Example 5

In 945 g of a middle chain fatty acid triglyceride and 5 g of the Polysorbate was dissolved 50 g of the compound of the invention. 10 g of the resulting solution was adsorbed into a premix of 20 g of calcium silicate and 40 g of calcium hydrogenphosphate and then 24 g of lactose was added to prepare a powdery mixture. 1 g of magnesium stearate and 5 g of talc were further added, and the mixture was uniformly admixed and then filled into capsules to prepare capsules.

### Example 6

In 945 g of a middle chain fatty acid triglyceride and 5 g of the Polysorbate was dissolved 50 g of the compound of the invention. 10 g of the resulting solution was adsorbed into a premix of 20 g of calcium silicate and 40 g of calcium hydrogenphosphate, and then 24 g of crystalline cellulose and 10 g of carmellose were added to prepare a powdery mixture. 1 g of magnesium stearate and 5 g of talc were further added, and the mixture was uniformly admixed and then tableted by means of a tableting machine to prepare tablets.

### Example 7

### Formulation (per one capsule)

| | |
|---|---|
| The compound of the invention | 30 mg |
| Oleic acid | 268.65 mg |
| Polysorbate 80 | 1.35 mg |
| Total | 300 mg |

In the oleic acid and the polysorbate was dissolved the compound of the invention to prepare a solution for encapsulation.

## Claims

1. An agent for preventing restenosis after coronary intervention therapy which comprises as an active ingredient a compound represented by the formula
